# EUROPÄISCHE PATENTANMELDUNG

(11) **EP 4 386 411 A1**
(43) Veröffentlichungstag der Anmeldung: **19.06.2024**
(21) Anmeldenummer: 22213380.3
(22) Anmeldetag: 14.12.2022
(51) Int. Cl.: G01R 33/28, G01R 33/31, F24F 7/007

(54) **MAGNETRESONANZTOMOGRAPHIESYSTEM UND VERWENDUNG EINES LUFTMENGENVERSTÄRKERS SOWIE EINES ADAPTIERTEN LÜFTERS**

(71) Anmelder: Siemens Healthineers AG, 91301 Forchheim (DE)
(72) Erfinder: Seegerer, Georg, 91056 Erlangen (DE)
(74) Vertreter: Siemens Healthineers Patent Attorneys

(57) **Zusammenfassung**

Magnetresonanztomographiesystem umfassend einen adaptierten Lüfter mit einem Luftausgang (5), wobei der adaptierte Lüfter einen Luftstromerzeuger (2), insbesondere mit einem Motor (3), einen Luftmengenverstärker (4), der an dem Luftausgang (5) angeordnet ist und/oder der den Luftausgang (5) umfasst, und einen Luftleitungsabschnitt (10), der einen durch den Luftstromerzeuger (2) erzeugten Luftstrom zu dem Luftmengenverstärker (4) leitet, umfasst, wobei der Luftmengenverstärker (4) derart ausgestaltet ist, dass er eine Luftmenge des über den Luftleitungsabschnitt (10) zu dem Luftmengenverstärker (4) geleiteten Luftstroms vergrößert, sodass er einen Luftstrom mit vergrößerter Luftmenge ausgibt.

## Beschreibung

Die Erfindung betrifft ein Magnetresonanztomographiesystem und eine Verwendung eines Luftmengenverstärkers sowie eines adaptierten Lüfters in und/oder an einem Magnetresonanztomographen.

Unabhängig vom grammatikalischen Geschlecht eines bestimmten Begriffes sind Personen mit männlicher, weiblicher oder anderer Geschlechteridentität mit umfasst.

Während des Betriebs eines Magnetresonanztomographen (MRT) wird in der Regel ein starkes magnetisches Feld im Inneren eines Untersuchungstunnels des MRT erzeugt. Dabei herrscht auch in der näheren Umgebung des MRT ein nicht unwesentliches magnetisches Streufeld, typischerweise in der Größenordnung von 10 mT bis 100 mT, vor. Dieses magnetische Streufeld kann störend für den Einsatz von elektrischen oder mechanischen Komponenten sein, insbesondere von elektrischen Motoren. Das Funktionsprinzip der Motoren basiert üblicherweise auf der Verwendung von Magnetfeldern und von Jochen aus Weicheisen, d.h. diese sind magnetisch weich. Die Motoren werden deshalb meist durch externe Magnetfelder, welche größer als 10 oder 20 mT sind, in ihrer Funktionsweise beeinträchtigt bzw. funktionieren teils gar nicht mehr. Beispielsweise beeinflusst ein externes Magnetfeld Eisenbleche des Läufers und Stators des Motors, so dass der Motor, selbst wenn er grundsätzlich noch funktioniert, oftmals höhere Verluste und eine geringere Leistung aufweist als ohne das externe Magnetfeld. Andererseits ist es jedoch wünschenswert, gerade in dem Bereich in der unmittelbaren Umgebung des MRT elektrische und mechanische Komponenten einzusetzen, weil dadurch der Aufbau bzw. die Montage des MRT an seinem Einsatzort vereinfacht werden kann und andererseits eine platzsparende Aufstellung möglich ist.

Elektrische Motoren werden unter anderem für Lüfter benötigt, welche z.B. für eine Zufuhr von Frischluft für eine Patienten in dem Untersuchungstunnel und für die Kühlung von (elektronischen) Komponenten bzw. elektrischen Schaltungen benötigt werden. Die Zufuhr von Frischluft in den Untersuchungstunnel hat einerseits den Vorteil, dass der Patient mit ausreichend frischer Luft versorgt werden kann, wodurch ein Gefühl einer stehenden und stickigen Luft, welches klaustrophobische Gefühle verstärken kann, vermieden wird. Weiterhin kann eine Kühlung des Patienten mit einem leichten Luftstrom kühler Luft es ermöglichen, mit Hochfrequenzpulsen an die erlaubte obere Leistungsgrenze zu gehen, sodass die mögliche obere Leistungsgrenze im Wesentlichen nur durch die erlaubte SAR *("specific absorption rate")* des Körpergewebes begrenzt ist. Dadurch kann eine bessere Bildqualität erreicht und gegebenenfalls auch die Scanzeit verkürzt werden. Schließlich ist mit einem Luftstrom üblicherweise eine effektivere Kühlung der sich im Betrieb erwärmenden Komponenten möglich als beispielsweise durch rein natürliche Konvektion. Elektronische Komponenten sind beispielsweise im Patiententisch verbaut. Diese sitzen daher nahe am Untersuchungsbereich bzw. dem *Field of View* des MRT und sind somit der vollen Magnetfeldstärke des MRT ausgesetzt (z.B. 0,5-3T für übliche Geräte).

Aufgrund der Tatsache, dass Motoren empfindlich für den Einfluss externer Magnetfelder sind, ist es somit erforderlich bei der Positionierung der Lüftermotoren nicht nur darauf zu achten, wo ein Luftstrom benötigt wird, sondern auch zu berücksichtigen, an welchen Stellen das magnetische Streufeld besonders niedrig ist.

Bisher ist es im Stand der Technik beispielsweise üblich, diesem Problem zu begegnen, indem durch Ausprobieren verschiedener Motoren an verschiedenen Positionen eine funktionsfähige Anordnung von Motoren erreicht wird. Dabei werden die Motoren insbesondere an Positionen in der näheren Umgebung des MRT positioniert, an denen das Streufeld eine etwas geringere Größe aufweist. Hierbei kommt es jedoch oftmals zu einer Konkurrenz um den zur Verfügung stehenden Platz bzw. Bauraum für verschiedene benötigte Motoren und auch gegenüber anderen Komponenten, die empfindlich auf magnetische Streufelder reagieren. Diese Lösung ist daher mit einem erhöhten Testaufwand verbunden, zumal zunächst oftmals nicht von Anfang an bekannt ist, welche Komponenten besonders empfindlich auf externe Streufelder reagieren. Diese Problematik bedingt die Notwendigkeit, in der Entwicklung mehrere Einbausituationen zu testen, um sicherzustellen, dass in einer Serienproduktion eine zuverlässige Funktionalität gegeben ist. Hinsichtlich der eingesetzten Lüfter müssen weiterhin oft Einschränkungen in Kauf genommen werden, z.B. dass weniger Lüfter, als optimal wäre, eingesetzt werden müssen oder dass leistungsschwächere Lüfter oder weniger günstig positionierte Lüfter eingesetzt werden müssen.

Eine weitere Möglichkeit, insbesondere, wenn man für einen Motor keinen Bauraum mit einem ausreichend niedrigen Streufeld finden kann, besteht darin, den Motor mit Gehäusen aus Eisenblech vom externen Magnetfeld abzuschirmen. Solche Gehäuse aus Eisenblech haben jedoch ein hohes Gewicht und verursachen zudem im magnetischen Streufeld hohe mechanische Kräfte.

Schließlich können Lüftermotoren auch weit weg von dem MRT bzw. dem MR-Magneten positioniert werden, d.h. beispielsweise in einer Entfernung von mehr als einem Meter und/oder in einem Nebenraum, in dem sich der MR-Magneten nicht befindet. In diesem Fall kann ein Luftstrom beispielsweise per Schlauch- oder Rohrleitung zu den gewünschten Stellen geleitet werden. Dies hat jedoch den Nachteil, dass dafür Schläuche mit großem Durchmesser verwendet werden müssen muss (oftmals 10cm Durchmesser oder mehr). Das Verwenden derart breiter Schläuche kostet zusätzliche Zeit bei der Installation und benötigt zusätzlichen Platz, an dem die Schläuche verlegt werden. Beispielsweise wird für ein Patientenlüfter, der für Frischluft im Behandlungstunnel verwendet wird, ein Schlauch mit nach Möglichkeit zumindest 50 mm Durchmesser eingesetzt, welcher einen Luftstrom zu einem Luftaustritt an einem Ende des Behandlungstunnels transportiert. Der Platz zwischen dem Plastikcover des MRT und dem Magneten ist jedoch begrenzt, sodass mitunter ein Schlauch mit einem etwas kleinerem Durchmesser verwendet werden muss. Dies führt dazu, dass ausgleichsweise ein signifikant stärkerer Lüftermotor verwendet werden muss, um einen benötigten Luftdurchsatz zu erreichen. Teilweise ist es bei üblichen Designs des Magneten und des Plastikcovers aus Platzgründen und wegen recht großer magnetischer Streufelder nicht möglich, dass der Lüfter direkt an einem gewünschten Ort des Luftaustritts sitzt.

Elektronische Komponenten, welche im oder am Untersuchungsbereich, z.B. am Patiententisch, angeordnet sind, können hingegen generell nur mit einer so geringen wärmeerzeugenden Verlustleistung eingeplant werden, wie per natürlicher Konvektion abgeführt werden kann. Dadurch müssen oftmals einschränkende Kompromisse, z.B. hinsichtlich der Qualität und Zuverlässigkeit der Komponenten bzw. der Messergebnisse, eingegangen werden.

Es ist daher eine Aufgabe der vorliegenden Erfindung, ein Magnetresonanztomographiesystem bereitzustellen, bei dem die oben genannten Probleme besser gelöst werden, bei dem insbesondere eine Lüftung trotz vorhandener magnetischer Streufelder positionell flexibler eingeplant werden kann.

Diese Aufgabe wird gelöst durch ein Magnetresonanztomographiesystem gemäß Anspruch 1, eine Verwendung eines Luftmengenverstärkers gemäß Anspruch 14 und eine Verwendung eines adaptierten Lüfters gemäß Anspruch 15. Weitere Vorteile und Merkmale ergeben sich aus den abhängigen Ansprüchen, der Beschreibung und den beigefügten Figuren.

Gemäß einem ersten Aspekt der Erfindung ist ein Magnetresonanztomographiesystem (MRT-System) umfassend einen adaptierten Lüfter mit einem Luftausgang vorgesehen, wobei der adaptierte Lüfter einen Luftstromerzeuger, insbesondere mit einem Motor, einen Luftmengenverstärker, der an dem Luftausgang angeordnet ist und/oder der den Luftausgang umfasst, und einen Luftleitungsabschnitt, der einen durch den Luftstromerzeuger erzeugten Luftstrom zu dem Luftmengenverstärker leitet, umfasst, wobei der Luftmengenverstärker derart ausgestaltet ist, dass er eine Luftmenge des über den Luftleitungsabschnitt zu dem Luftmengenverstärker geleiteten Luftstroms vergrößert, sodass er einen Luftstrom mit vergrößerter Luftmenge ausgibt. Der Begriff "adaptierter Lüfter" zielt darauf ab, dass der Lüfter an den Umstand angepasst ist, dass während des Betriebs durch das MRT erzeugte magnetische Streufelder in der Umgebung des MRT vorliegen. Insbesondere ist der Lüfter dadurch adaptiert, dass er den Luftmengenverstärker umfasst, der an dem Luftausgang angeordnet ist bzw. den Luftausgang umfasst. Der Luftmengenverstärker kann insbesondere derart ausgestaltet sein, dass er eine aerodynamische Ausgestaltung bzw. ein aerodynamisches System umfasst, wodurch der Luftstrom derart aus dem Luftausgang des Luftmengenverstärker austritt, dass Umgebungsluft mitgerissen wird und damit die Luftmenge verstärkt. Die Ausgestaltung des Luftmengenverstärkers und/oder die Anbindung des Luftleitungsabschnitts an den Luftmengenverstärker, optional auch die Ausgestaltung des Luftstromerzeugers, kann beispielsweise entsprechend wie beschrieben in dem Dokument US 2009/060710 A1 vorgesehen sein. Der Luftmengenverstärker kann insbesondere als ein "Bladeless Fan" ausgebildet sein. Vorzugsweise kann der Luftmengenverstärker selbst den Luftausgang des adaptierten Lüfters umfassen, bzw. der Luftausgang des Luftmengenverstärkers kann dem Luftausgang des adaptierten Lüfters entsprechen. Es ist alternativ auch denkbar, dass in Strömungsrichtung hinter dem Luftmengenverstärker noch ein separater Luftausgang des adaptierten Lüfters vorgesehen ist, insbesondere zusätzlich zu dem Luftausgang des Luftmengenverstärkers. In diesem Fall kann der Luftmengenverstärker an dem Luftausgang des adaptierten Lüfters angeordnet sein. Ein separater Luftausgang kann beispielsweise dazu ausgestaltet sein, den Luftstrom mit verstärkter Luftmenge umzulenken, zu fokussieren oder auf andere Weise zu beeinflussen. Der Luftstrom mit vergrößerter Luftmenge, welcher dadurch erzeugt wird, kann insbesondere eine geringere Geschwindigkeit aufweisen als der zu dem Luftmengenverstärker geleitete Luftstrom.

Der Luftstromerzeuger kann beispielsweise auf dem Prinzip eines Lüfters mit durch den Motor angetriebenen Rotorblättern basieren, wobei die Rotorblätter insbesondere so ausgestaltet sind, dass sie, wenn sie durch den Motor betrieben werden, den Luftstrom erzeugen, der über den Luftleitungsabschnitt zu dem Luftmengenverstärker geleitet wird. Der Luftleitungsabschnitt kann beispielsweise ein Luftleitungsschlauch oder ein Luftleitungsrohr sein. Durch den Luftleitungsabschnitt ist es möglich, den Luftstromerzeuger separat von dem Luftmengenverstärker anzuordnen. Insbesondere kann der Luftstromerzeuger, und insbesondere der Motor des Luftstromerzeugers, an einer Position angeordnet sein, welche während eines Betriebs des MRT einem weniger starken Magnetfeld ausgesetzt ist als die Position, in der der Luftmengenverstärker angeordnet ist. Mit anderen Worten kann eine räumliche Trennung von dem Motor und dem Luftausgang erreicht werden. Vorteilhafterweise kann somit beim Design des MRT-System leichter erreicht werden, dass der Motor und auch der der gesamte Luftmengenerzeuger mit seinen möglicherweise beweglichen Teilen, z.B. Rotorblättern, an einem Ort mit geringer Magnetfeldstärke angeordnet wird, während gleichzeitig nur eine geringere Luftmenge von dem Luftstromerzeuger zu dem Luftausgang transportiert werden muss. Müsste hingegen eine größere Luftmenge, insbesondere die komplette am Luftausgang ausgegebenen Luftmenge über den Luftleitungsabschnitt transportiert werden, so würde dies zu deutlich höheren Druck- und Strömungsverlusten führen. Eine erhöhte Luftmenge, welche pro Zeiteinheit befördert werden soll, führt zu einer erhöhten Strömungsgeschwindigkeit. Bei angenommener laminarer Strömung in dem Luftleitungsabschnitt führt diese erhöhte Strömungsgeschwindigkeit zu Strömungsverlusten welcher quadratisch mit der Geschwindigkeit ansteigen. Aufgrund der geringeren Strömungsverluste kann mit dem erfindungsgemäßen MRT-System folglich ein weniger leistungsstarker Motor verwendet werden, womit dieser sowohl in der Anschaffung als auch im Betrieb kostengünstiger sein kann. Andererseits besteht auch die Option, den Durchmesser des Luftleitungsabschnitts zu verringern, wodurch weniger Bauraum benötigt wird und folglich weniger Kompromisse bei der Gestaltung des MRT-Systems eingegangen werden müssen. Auch kann ein längerer Luftleitungsabschnitt verwendet werden, wodurch der Motor in einem Bereich mit geringerer magnetischer Feldstärke angeordnet werden kann, insbesondere in einem Bereich, welcher weiter von dem Magneten des MRT und/oder von dem Ort des Luftausgangs gelegen ist.

Nach einer bevorzugten Ausführungsform ist der Luftmengenverstärker aus einem nicht-magnetischen bzw. nicht-magnetisierbaren Material und/oder einem nicht-leitfähigen Material, insbesondere aus einem Kunststoff, gefertigt. Vorteilhafterweise kann somit erreicht werden, dass die physikalische Funktionsweise des MRT, welche starke Magnetfelder und elektromagnetische Hochfrequenz-Impulse benötigt, nicht wesentlich durch eine Interaktion mit magnetisierbaren Komponenten des Luftmengenverstärkers gestört wird. Beispielsweise könnten ansonsten durch Gradientenimpulse des MRT in leitfähige Bauteile des Luftmengenverstärkers elektrische Ströme induziert werden. Auch kann mit dieser Ausführungsform erreicht werden, dass keine bzw. nur vernachlässigbar geringe magnetische Kräfte auf den Luftmengenverstärker wirken. Mit dieser Ausführungsform kann eine Platzierung nahe am oder im Untersuchungsbereich bzw. *Field of View* ermöglicht werden. "Nahe" kann in diesem Kontext insbesondere bedeuten, dass ein Abstand von weniger als 50 cm vorgesehen ist. Beispielsweise kann es denkbar sein, den Luftausgang direkt in Innenbereich eines Untersuchungstunnels des MRT vorzusehen, in welchem beim Betrieb des MRT die volle Feldstärke des erzeugten Magnetfelds vorherrscht.

Gemäß einer Ausführungsform ist der Luftmengenverstärker derart ausgestaltet, dass er durch seine geometrische Gestaltung die Luftmenge passiv vergrößert. Der Begriff passiv ist dabei insbesondere derart zu verstehen, dass die Luftmenge ohne aktive Bewegung einzelner Komponenten des Luftmengenverstärkers und/oder ohne aktiven Antrieb des Luftmengenverstärkers vergrößert wird. Insbesondere umfasst und benötigt der Luftmengenverstärker keinen eigenen Motor. Durch die passive Gestaltung, insbesondere durch das Vermeiden von beweglichen Komponenten und/oder durch das Fehlen eines Motors, kann vorteilhafterweise eine Interaktion mit dem Magnetfeld des MRT verringert werden. Insbesondere kann damit eine Induktion von durch das Magnetfeld erzeugten Wirbelströmen verhindert werden. Der Luftausgang des Luftmengenverstärkers kann derart ausgestaltet sein, dass er eine konvexe Figur vollständig oder teilweise umschließt. Dabei wird insbesondere von einer Blickrichtung entgegen der Richtung des ausströmenden Luftstroms mit vergrößerter Luftmenge ausgegangen. Der Luftausgang kann vorzugsweise gemäß einem vollständigen oder teilweise geöffneten Oval oder Vieleck ausgestaltet sein. Das Oval kann insbesondere eine Ellipse oder ein Kreis sein. Das Vieleck kann vorzugsweise ein Viereck, insbesondere Rechteck, sein, wobei die Ecken des Vierecks optional abgerundet sein können. Ein teilweises Umschließen bzw. geöffnet sein beinhaltet dabei vorzugsweise ein Umschließen bzw. geöffnet sein zu mehr als 50% und weniger als 95%, insbesondere durch einen zusammenhängenden Luftausgang. Damit kann eine besonders effiziente Luftverstärkung erreicht werden. Die Form des Luftausgangs kann an die geometrischen Gegebenheiten des jeweiligen Einsatzbereichs des Luftmengenverstärkers and dem MRT-System angepasst sein. Es kann vorgesehen sein, dass die Anbindung des Luftleitungsabschnitts an den Luftausgang seitlich an dem Luftausgang bzw. seitlich an der konvexen Figur, insbesondere im Wesentlichen senkrecht zu der Fläche der konvexen Figur, angeordnet und/oder befestigt ist. "Im Wesentlichen" bedeutet dabei insbesondere mit einer Winkelabweichung von nicht mehr als 15°. Dabei ist die konvexe Figur bzw. die Fläche, welche durch den Luftausgang vollständig oder teilweise umschlossen wird, insbesondere sowohl zu ihrer Vorderseite und auch zu ihrer Rückseite hin offen. Der Luftausgang ist vorzugsweise derart ausgestaltet, dass beim Ausströmen der Luft eine Druckdifferenz zwischen den beiden Seiten der konvexen Figur und/oder zwischen der Seite, in welche der verstärkte Luftstrom hinströmt, und der dazu entgegengesetzten Seite erzeugt wird. Vorteilhafterweise kann dadurch Umgebungsluft mit dem ausgegebenen Luftstrom mitgezogen werden, wodurch der Luftstrom verstärkt werden kann. Vorzugsweise kann die Rückseite des Luftausgangs, insbesondere in einer Querschnittsansicht senkrecht zu der Strömungsrichtung des ausströmenden Luftstroms abgerundet bzw. gekurvt ausgebildet sein. Insbesondere kann die Querschnittsansicht in ihrem Außenumfang an den Querschnitt des Tragflügels eines Flugzeugs angelehnt sein. Dabei kann vorzugsweise der Luftausgang an dem dickeren Ende des Querschnitts angeordnet sein und in eine Richtung weisen, die von der Richtung des sich verjüngenden Teils um einen Winkel von 5° bis 60° zu einem Innenbereich der konvexen Figur hin abweicht. Der detaillierte Aufbau des Luftmengenverstärkers kann grundsätzlich analog zu im Stand der Technik bekannten Prinzipien vorgesehen sein. Diese sind zum Beispiel in der Veröffentlichung von Mohammad Jafari et al.: "Numerical investigation of geometric parameter effects on the aerodynamic performance of a Bladeless fan", Alexandria Engineering Journal (2016) 55, 223-233; http://dx.doi.org/10.1016/j.aej.2015.11.001 und in der Veröffentlichugn von Anutha M A et al.: "Design Development and Analysis of Bladeless Thruster", International Journal of Electrical, Electronics and Data Communication, ISSN(p): 2320-2084, ISSN(e):2321-2950, Volume 8, Issue 6, Jun. 2020 beschrieben und erklärt.

Gemäß einer Ausführungsform ist der von dem Luftmengenverstärker umfasste Luftausgang ringförmig oder in Form eines Ringabschnitts ausgestaltet. Zusätzlich oder alternativ weist der Luftmengenverstärker einen luftführenden Ring oder Ringabschnitt mit Luftausgangsöffnungen auf, durch welche Luft in eine Richtung ausgegeben wird, die eine Richtungskomponente senkrecht zu der Ringebene aufweist, insbesondere in eine Richtung senkrecht zur Ringebene oder in eine um maximal 30° von der Richtung senkrecht zur Ringebene abweichenden Richtung ausgegeben wird. Die Luftausgangsöffnungen können auch zu einer gemeinsamen entlang dem Ring langgezogenen Öffnung kombiniert sein. Der Ring muss dabei nicht notwendigerweise kreisförmig ausgestaltet sein, sondern kann allgemein oval oder ellipsenförmig ausgestaltet sein. Eine Gestaltung als Ring oder Ringabschnitt kann besonders günstig für das Erzielen einer Luftverstärkung sein. Insbesondere umschließt der Ring oder Ringabschnitt eine konvexe Figur vollständig oder teilweise, wie oben beschrieben.

Gemäß einer Ausführungsform ist der Luftstromerzeuger in einem ersten Bereich angeordnet, der, wenn das Magnetresonanztomographiesystem während des Betriebs ein Magnetfeld erzeugt, eine geringere magnetische Feldstärke aufweist als ein zweiter Bereich, in dem der Luftmengenverstärker angeordnet ist. Mit dieser Ausführungsform kann der Vorteil, dass mit dem erfindungsgemäßen MRT-System aufgrund der geringen durch den Luftleitungsabschnitt zu befördernden Luftmenge eine flexible Anordnung des Luftstromerzeugers bzw. des Motors erzielt werden kann, ausgenutzt werden, indem die Anfälligkeit, die Motoren üblicherweise für externe Magnetfelder haben, durch eine entsprechende Positionierung kompensiert werden kann. Vorzugsweise kann der erste Bereich einen Abstand von zumindest 1m zu dem Hauptmagneten des MRT aufweisen und/oder in einem Bereich des MRT-Systems angeordnet bzw. befestigt sein, der eine geringere magnetische Feldstärke aufweist als die gesamten außerhalb des Hauptmagneten gelegenen Bereiche des MRT-Systems, die weniger als 1m von dem Hauptmagneten beabstandet sind, im Durchschnitt. Beispielsweise kann es vorgesehen sein, dass sich der erste Bereich in einem anderen Raum als der Hauptmagnet und/oder der Untersuchungstunnel des MRT befindet. Alternativ oder zusätzlich kann der erste Bereich in einem in Längsrichtung des Untersuchungstunnels gesehen mittleren Bereich seitlich und außerhalb von dem Hauptmagneten angeordnet sein, insbesondere in der Nähe der Außenwand und/oder an einer Außenwand des MRT. Der mittlere Bereich kann insbesondere der Bereich sein, welcher in Längsrichtung des Untersuchungstunnels gesehen im mittleren Drittel des Untersuchungstunnels gelegen ist. Die Außenwand kann insbesondere eine Seitenwand und/oder eine Bodenwand des MRT sein. "In der Nähe der Außenwand" kann insbesondere bedeuten, dass der erste Bereich maximal einen Abstand zu der Außenwand aufweist der nicht größer ist als der in Längsrichtung des Untersuchungstunnels gesehene Abstand zu dem vorderen oder hinteren Ende des Untersuchungstunnels. Vorteilhafterweise ist das magnetische Streufeld des MRT-Systems in der Regel in dem mittleren Bereich bzw. mittleren Drittel geringer als an anderen Orten seitlich des Hauptmagneten. Alternativ oder zusätzlich kann der mittlere Bereich in einem unteren Fußraum des MRT angeordnet sein. Durch die ermöglichte günstigere Positionierung des Motors kann insbesondere die Wahl des Motors weniger restriktiv vorgenommen werden, indem weniger stark darauf geachtet werden muss, dass der Motor resistent gegen externe Magnetfelder ist.

Gemäß einer Ausführungsform ist der Luftmengenverstärker dazu ausgestaltet, die Luftmenge um zumindest das 5-fache, bevorzugt zumindest das 10-fache, besonders bevorzugt zumindest das 15-fache, zu verstärken. Die Möglichkeit, eine derartige Verstärkung zu realisieren wurde von Mohammad Jafari et al. in "Numerical investigation of geometric parameter effects on the aerodynamic performance of a Bladeless fan", Alexandria Engineering Journal (2016) 55, 223-233 aufgezeigt. Vorteilhafterweise kann es somit vorgesehen sein, durch eine Verstärkung der Luftmenge direkt am Ort, an dem der Luftstrom benötigt wird, d.h. insbesondere am bzw. vor dem Luftausgang, die Luftmenge, die durch den Luftleitungsabschnitt geleitet wird, deutlich zu verringern. Dadurch kann beispielsweise ein Luftleitungsabschnitt mit geringerem Durchmesser verwendet werden. Beispielsweise ist es bei einer Verstärkung auf zumindest das 10-fache denkbar, sowohl einen Luftleitungsabschnitt mit geringerem Durchmesser zu verwenden, als auch einen Motor mit geringerer Leistung zu betreiben, da die Strömungsverluste bei geringerer geleiteter Luftmenge geringer sind. Es hat sich ergeben, dass bei einer Reduzierung der transportierten Luftmenge auf 1/15, was bei einer danach vorgesehenen Verstärkung auf das 15-fache möglich ist, auf schätzungsweise ca. 1/225 reduziert werden kann. Beispielsweise kann somit alternativ oder zusätzlich ein längerer Luftleitungsabschnitt vorgesehen werden. Dadurch erhöhen sich die Freiheiten bzw. Wahlmöglichkeiten bei der Gestaltung und Konstruktion des MRT-Systems. Bei einer 15-fachen Verstärkung hat sich ergeben, dass zur Kompensation der Druck der Luftmenge, die durch den Luftleitungsabschnitt geleitet wird, um etwa den Faktor 5 höher sein muss als wenn die Luftmenge direkt in größerer Menge zum Luftausgang geleitet wird, um eine passive Verstärkung zu ermöglichen. Dennoch wiegen die Vorteile, die sich durch die geringeren Reibungsverluste ergeben und die quadratisch in die Energiebilanz eingehen, in der Regel deutlich stärker als diese Einschränkung. Beispielsweise kann es vorgesehen sein eine Luftverstärkung von maximal dem 30-fachen vorzusehen, insbesondere um den benötigten höheren Druck einzuschränken.

Gemäß einer Ausführungsform ist der Luftstromerzeuger dazu ausgestaltet, den Luftstrom in den Luftleitungsabschnitt mit einem Druck von 80 bis 500 Pa, bevorzugt, 100 bis 350 Pa, besonders bevorzugt von 150 Pa bis 300 Pa einzugeben. Arbeitet der Luftmengenverstärker gemäß einer bevorzugten passiv, so muss sich die benötigte Energie für die Verstärkung des Luftstroms aus der Energie durch den Luftleitungsabschnitt zugeleiteten Luftstroms ergeben. Dies kann insbesondere erfolgen, indem der durch den Luftleitungsabschnitt zugeleitete Luftstrom einen höheren Druck aufweist, als ein entsprechender Luftstrom, der nicht verstärkt werden muss. Durch den erhöhten Druck kann der vorhandene Luftstrom genügend Energie aufweisen, um während der Luftverstärkung ausreichend Umgebungsluft mitzureißen und damit den Luftstrom zu verstärken. Ein Druck von 100 bis 350 Pa kann dabei verhältnismäßig problemlos durch gängige Motoren bzw. Lüfter erzeugt werden, insbesondere, da eine geringere Luftmenge verwendet werden kann. Insbesondere bei einem erzeugten Druck von 150 Pa bis 300 Pa kann, aufgrund der damit möglichen Verstärkung, die verwendete Luftmenge so deutlich verringert werden, dass nur ein ausreichend geringer Bruchteil der am Luftausgang benötigten Luftmenge durch den Luftleitungsabschnitt geleitet werden muss, womit insbesondere Strömungsverluste erheblich reduziert werden können. Die vorgegebenen Druckbereiche sind dabei nicht so zu verstehen, dass die Motoren nicht auch zusätzlich in der Lage sein können, optional geringere Drücke einzugeben. Beispielsweise können die Motoren herunterregelbar sein. Dies kann z.B. zweckmäßig sein, wenn gerade nur eine geringere Kühlleistung benötigt wird, insbesondere im Zusammenhang mit einer sich automatisch einstellenden Kühlleistung.

Gemäß einer Ausführungsform ist der Luftstromerzeuger ein Diagonallüfter oder Radiallüfter bzw. ist als Diagonallüfter oder Radiallüfter ausgebildet. Diagonallüfter und Radiallüfter sind insbesondere besonders geeignet für das Erzeugen eines hohen Differenzialdrucks bei gleichzeitig geringerem Luftdurchsatz. Sie können damit besonders geeignet sein für den erfindungsgemäßen adaptierten Lüfter, wenn insbesondere nur eine verringerte Luftmenge durch den Luftleitungsabschnitt geleitet wird.

Gemäß einer Ausführungsform umfasst das Magnetresonanztomographiesystem einen Untersuchungstunnel mit einem Tunneleingang, wobei der Luftmengenverstärker an dem Tunneleingang angeordnet ist und so ausgerichtet ist, dass der verstärkte Luftstrom in den Untersuchungstunnel, insbesondere im Wesentlichen entlang der Längsachse des Untersuchungstunnels, gerichtet ist, oder so ausgerichtet ist, dass der verstärkte Luftstrom aus dem Untersuchungstunnel herausgerichtet ist. Der Tunneleingang kann insbesondere ein hinterer Tunneleingang sein, gegenüber dem Eingang, an dem eine Patientenliege angeordnet ist und/oder an dem ein Patient eingeführt wird, und/oder der Tunneleingang kann ein vorderer Tunneleingang sein, welcher dem Eingang, an dem eine Patientenliege angeordnet ist und/oder an dem ein Patient eingeführt wird, entspricht. Der Tunneleingang kann (temporär) durch ein Verschlussmittel geschlossen sein und dennoch als Tunneleingang zu bezeichnen sein. Mit dieser Ausführungsform kann es ermöglicht werden, einen Patienten mit ausreichend frischer Luft zu versorgen. Eine Anordnung an einem hinteren Tunneleingang kann es vorteilhafterweise ermöglichen, dass gegebenenfalls ein Einführen des Patienten weniger behindert wird und weitere Komponenten, welche bevorzugt an einem vorderen Tunneleingang platziert werden (z.B. ein Display, ein Bedien-Panel, ein Kreuzlaser für eine Positionierung, eine Mechanik für das Andocken einer Patientenliege, etc.) ggf. den Platz an dem vorderen Tunneleingang benötigen. Eine Anordnung an einem vorderen Tunneleingang kann den Vorteil haben, dass eventuell eine Wartung oder ein Einstellen des Luftstromverstärkers leichter bzw. schneller vorgenommen werden kann. Eine Ausrichtung des Luftstroms in den Untersuchungstunnel kann den Vorteil haben, dass der gesamte verstärkte Luftstrom für die Lüftung verwendet werden kann. Eine Ausrichtung des Luftstroms aus dem Untersuchungstunnel heraus kann den Vorteil haben, dass ein (geringerer) Luftstrom aus dem Tunnel gesaugt wird (entsprechend der durch die Luftstromverstärkung hinzugefügte Menge an Luft), sodass immer noch ein Lüftungseffekt in dem Untersuchungstunnel erzielt werden kann. Gegebenenfalls kann somit auch eine schnellere Luftströmung direkt in das Gesicht eines Patienten verhindert werden.

Mit dem erfindungsgemäßen adaptierten Lüfter kann dies ermöglicht werden, wobei gleichzeitig der Motor des Lüfters weiter entfernt von dem Tunneleingang platziert sein kann. Dies ist vorteilhaft, weil einerseits am Tunneleingang bzw. in der Nähe des Tunneleingangs wenig verfügbarer Platz ist, insbesondere da dort auch noch andere Komponenten, z.B. zumindest eine Gradientenspule, platziert werden müssen. Andererseits ist das dortige Magnetfeld während des Betriebs des MRT in der Regel sehr stark bzw. zu stark für den Betrieb üblicher Motoren. Vorzugsweise kann der Luftmengenverstärker innerhalb des Untersuchungstunnels am Tunneleingang angeordnet sein.

Gemäß einer Ausführungsform ist der von dem Luftmengenverstärker umfasste Luftausgang entlang einem Rand des Tunneleingangs angeordnet und umgibt insbesondere den Tunneleingang ringförmig oder teilringförmig. Insbesondere kann der von dem Luftmengenverstärker umfasste Luftausgang ringförmig oder in Form eines Ringabschnitts ausgestaltet sein. Vorzugsweise ist die Form des Luftausgangs bzw. die Ringform des Luftmengenverstärkers an die Form des Tunneleingangs angepasst. Der teilringförmige bzw. als Ringabschnitt ausgestaltete Luftausgang ist vorzugsweise zumindest halbringförmig besonders bevorzugt zumindest dreiviertelringförmig ausgebildet. Eine vollständige Ringform kann eine besonders effiziente Luftverstärkung und gleichzeitig eine besonders gleichmäßige Luftverteilung ermöglichen. Eine Teilringform kann vorteilhaft sein, um zu gewährleisten, dass ausreichend Platz für weitere Komponenten, z.B. Anschlussleitungen der Gradientenspule, vorhanden ist. Vorzugsweise bedeutet teilringförmig, dass sich die die Ringform über maximal 95%, besonders bevorzugt maximal 90%, einer vollständigen Ringform erstreckt. Mit dieser Ausführungsform kann die Form des Luftverstärkers bzw. des Luftausgangs des Luftverstärkers vorteilhafterweise an den Tunneleingang angepasst sein. Damit kann ein besonders gleichmäßiger Luftstrom erzeugt werden, was insbesondere für einen Patienten besonders angenehm sein kann. Dabei wird sich zunutze gemacht, dass eine ringförmiger bzw. an den Tunneleingang angepasster Luftverstärker gleichmäßig verteilt über den gesamten oder nahezu gesamten Tunnelquerschnitt einen Luftstrom erzeugen kann. Es kann vorgesehen insbesondere sein, dass die Anbindung des Luftleitungsabschnitts an den Luftausgang seitlich an dem Luftausgang angeordnet bzw. fixiert ist. Vorteilhafterweise kann damit erreicht werden, dass dadurch der Tunneleingang nicht wesentlich durch den adaptierten Lüfter blockiert wird. Durch den gleichmäßigen Luftstrom kann weiterhin erreicht werden, dass der Luftstrom nicht lokal unangenehm stark für den Patienten eingestellt werden muss. Dies spielt insbesondere deshalb eine Rolle, da der Patient, ja nach untersuchtem Körperbereich, an verschiedenen Positionen des Untersuchungstunnels platziert wird. Ein weniger gleichmäßiger Luftstrom, von Lüftern nach dem Stand der Technik, hat hierbei den Nachteil, dass er an manchen Stellen, insbesondere nahe dem Luftausgang, notwendigerweise besonders stark sein muss, um auch entferntere Stellen effektiv zu belüften.

Gemäß einer Ausführungsform umfasst das Magnetresonanztomographiesystem eine Patientenliege, die dazu ausgestaltet ist, zumindest teilweise in einen Untersuchungsbereich des Magnetresonanztomographiesystems bewegt zu werden, wobei der Luftmengenverstärker an der Patientenliege angeordnet und befestigt ist, sodass er, wenn die Patientenliege in den Untersuchungsbereich bewegt wird, mit der Patientenliege in den Untersuchungsbereich bewegt wird. Der Untersuchungsbereich kann sich insbesondere in dem Untersuchungstunnel des MRT befinden. Die Patientenliege ist insbesondere von einer Außenstelle außerhalb des Untersuchungstunnels in eine Untersuchungsstellung innerhalb des Untersuchungstunnels verfahrbar. Mit dieser Ausführungsform wird es ermöglicht, auch innerhalb des Untersuchungsbereichs einen Luftstrom auszugeben, z.B. um Komponenten in dem Untersuchungsbereich zu kühlen. Gemäß einer Ausführungsform umfasst die Patientenliege zumindest eine elektronische Komponente, insbesondere zumindest eine Empfangsspule, wobei der Luftmengenverstärker derart angeordnet ist, dass der ausgegebene verstärkte Luftstrom zu einem überwiegenden Teil, bevorzugt im Wesentlichen, auf die zumindest eine elektronische Komponente gerichtet ist. "Im Wesentlichen" kann in diesem Zusammenhang so zu verstehen sein, dass zumindest 85% des Luftstroms auf die Komponente gerichtet ist. Im Untersuchungsbereich herrscht üblicherweise während des Betriebs des MRT die volle Stärke des MRT-Magnetfelds vor, z.B. im Bereich von 0.5T-3T oder auch mehr. Ein Lüfter gemäß dem Stand der Technik kann hier üblicherweise nicht verwendet werden. Durch die hierin beschriebene Ausgestaltung des adaptierten Lüfters, insbesondere durch eine räumliche Trennung von Motor und Luftausgang, kann es jedoch vorteilhafterweise ermöglicht werden, auch in dem Untersuchungsbereich einen Luftstrom auszugeben. Durch die Luftverstärkung kann es dabei insbesondere erreicht werden, dass nur eine geringer Luftmenge zu dem Luftausgang geleitet werden muss.

Gemäß einer Ausführungsform umfasst das Magnetresonanztomographiesystem einen Untersuchungstunnel mit einem Tunneleingang, über den die Patientenliege in den Untersuchungsbereich bewegbar ist, wobei der Untersuchungsbereich in dem Untersuchungstunnel angeordnet ist, wobei das Magnetresonanztomographiesystem ein vor dem Tunneleingang angeordnetes Stützelement umfasst, wobei die Patientenliege durch Aufliegen auf dem Stützelement gestützt wird, soweit sie außerhalb des Untersuchungstunnel bewegt wird, wobei der Luftstromerzeuger außen an dem Stützelement angeordnet ist oder in das Stützelement integriert ist, wobei der Luftleitungsabschnitt zwischen dem Luftstromerzeuger und dem Luftmengenverstärker flexibel, insbesondere als Luftschlauch, ausgebildet ist. Vorzugsweise ist der Luftstromerzeuger an einem Fußende des Stützelements und/oder in einer von dem Tunneleingang abgewandten Hälfte des Stützelements angeordnet. An dem Fußende ist das magnetische Streufeld üblicherweise relativ niedrig, typischerweise in der Größenordnung von 5mT oder weniger. An dieser Position ist der Motor auch ausreichend weit von dem Untersuchungsbereich entfernt, um wesentliche Einflüsse auf eine Messung ausschließen zu können. Ein Motor in dem Untersuchungsbereich oder in der Nähe des Untersuchungsbereichs wäre hingegen nachteilig, weil sich bewegende Metallteile des Motors aufgrund ihrer Leitfähigkeit zu Wirbelströmen führen würden, die die Homogenität des Magnetfeldes stören und damit zu Bildfehlern führen können. Aufgrund der Luftverstärkung am Luftausgang, ist es möglich einen besonders dünnen Luftleitungsabschnitt bzw. Luftschlauch zu verwenden, wodurch nur relativ wenig Bauraum für die Umsetzung dieser Ausführungsform genügen kann. Beispielsweise kann ein Durchmesser des Luftleitungsabschnitts auf maximal 3 cm begrenzt sein. Mit diesem Durchmesser kann eine Integration in bestehende Systeme ermöglicht werden, ohne wesentliche Änderungen an dem Design der Mechanik und/oder der Elektronik der Patientenliege vornehmen zu müssen.

Ein weiterer Aspekt der Erfindung ist eine Verwendung eines Luftmengenverstärkers, insbesondere eines Luftmengenverstärkers wie hierin beschrieben, und/oder eines adaptierten Lüfters wie hierin beschrieben in und/oder an einem Magnetresonanztomographen, insbesondere zur Erzeugung eines verstärkten Luftstroms und/oder zur Kühlung zumindest einer Komponente des Magnetresonanztomographen. Alle Vorteile und Merkmale des Magnetresonanztomographiesystem können analog auf die Verwendung eines Luftmengenverstärkers übertragen werden und umgekehrt.

Ein weiterer Aspekt der Erfindung ist eine Verwendung eines adaptierten Lüfters, insbesondere eines adaptierten Lüfters wie hierin beschrieben, zur Kühlung zumindest einer Komponente eines Magnetresonanztomographen und/oder zur Belüftung eines Untersuchungsbereichs des Magnetresonanztomographen, wobei der adaptierte Lüfter einen Luftstromerzeuger mit einem Motor, einen Luftmengenverstärker, der an dem Luftausgang angeordnet ist und/oder der der Luftausgang ist, und einen Luftleitungsabschnitt, der einen durch den Luftstromerzeuger erzeugten Luftstrom zu dem Luftmengenverstärker leitet, umfasst, wobei der Luftmengenverstärker derart ausgestaltet ist, dass er eine Luftmenge des über den Luftleitungsabschnitt zu dem Luftmengenverstärker geleiteten Luftstroms vergrößert, sodass er einen Luftstrom mit vergrößerter Luftmenge ausgibt. Alle Vorteile und Merkmale des Magnetresonanztomographiesystem und der Verwendung eines Luftmengenverstärkers können analog auf die Verwendung eines adaptierten Lüfters übertragen werden und umgekehrt.

Alle hierin beschriebenen Ausführungsformen können miteinander kombiniert werden, soweit nicht explizit etwas anderes angegeben ist.

Im Folgenden werden Ausführungsformen mit Bezug auf die beigefügten Figuren beschrieben.
- Fig. 1: zeigt eine Perspektivansicht auf ein Magnetresonanztomographiesystem gemäß dem Stand der Technik,
- Fig. 2: zeigt eine Schnittansicht auf ein Magnetresonanztomographiesystem gemäß dem Stand der Technik,
- Fig. 3: zeigt eine Perspektivansicht auf ein Magnetresonanztomographiesystem gemäß einer Ausführungsform der Erfindung,
- Fig. 4: zeigt eine Schnittansicht auf ein Magnetresonanztomographiesystem gemäß einer Ausführungsform der Erfindung,
- Fig. 5: zeigt eine Schnittansicht auf ein Magnetresonanztomographiesystem mit ausgefahrener Patientenliege gemäß einer weiteren Ausführungsform der Erfindung,
- Fig. 6: zeigt eine Schnittansicht auf ein Magnetresonanztomographiesystem mit eingefahrener Patientenliege gemäß einer weiteren Ausführungsform der Erfindung, und
- Fig. 7: zeigt eine Ansicht auf einen adaptierten Lüfter gemäß einer Ausführungsform der Erfindung.

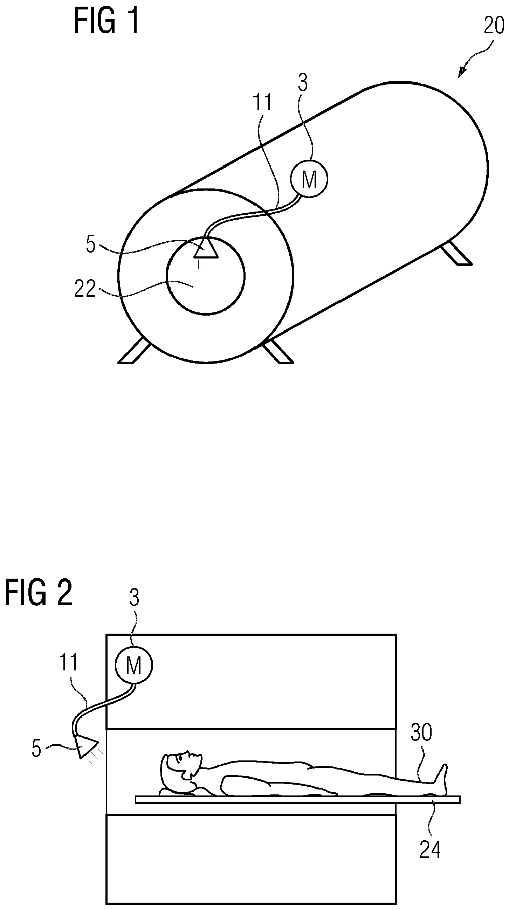

Die Figuren 1 und 2 zeigen ein Magnetresonanztomographiesystem gemäß dem Stand der Technik in einer Perspektivansicht (Figur 1) und in einer seitlichen Schnittansicht (Figur 2). Der Luftausgang 5 ist hierbei an einem hinteren Ende im oberen Bereich des Untersuchungstunnels 22 des Magnetresonanztomographen (MRT) 20, in dem sich der Untersuchungsbereich befindet, angeordnet. Dabei drückt ein Motor 3 Luft durch einen Schlauch 11 zu dem Luftausgang 5. Der Motor 3 ist außerhalb des MRT-Magneten an dem MRT angeordnet. Einerseits ist dabei die Wahl des Motors 3 hierbei eingeschränkt, denn der Motor muss einigermaßen resistent gegenüber externen Magnetfeldern sein. Andererseits ist auch die Positionierung des Motors 3 eingeschränkt, denn er muss an einem Ort mit möglichst geringer Magnetfeldstärke sein und gleichzeitig relativ nah an dem Luftausgang 5 positioniert sein, denn die Länge des Luftschlauchs 11 ist beschränkt, damit die gesamte benötigte Luftmenge noch durch den Luftschlauch 11 gelangen kann. Hinsichtlich der Positionierung gibt es insbesondere eine Konkurrenz mit anderen Komponenten. Hier ist der Motor oben seitlich am Magneten in der Nähe des hinteren Endes des Untersuchungstunnels 22 angeordnet. Der Motor 3 ist dabei üblicherweise außerhalb des MRT-Magneten aber noch innerhalb eines Plastik-Covers des MRT 20 platziert. Der Luftschlauch 11 weist von dem Anschluss am Motor 3 bis zum Luftausgang 5 eine Länge von etwa 50cm auf und hat einen Durchmesser von ca. 50-90mm. Der Motor 3 darf nicht direkt am Luftausgang 5 positioniert sein, weil dort erstens wenig Platz ist und dort zudem noch andere Komponenten platziert werden müssen (z.B. Gradientenspule). Zweitens herrscht ein zu starkes Streufeld des großen MRT-Magneten vor und drittens können magnetische Wechselfelder erzeugt werden, deren Interaktion mit dem Motor 3 sowohl den Motor 3 als auch MRT-Messungen stören würden. Wäre der Motor 3 zu nahe an dem Untersuchungsbereich positioniert, würden Wirbelströme in die metallischen Teile des Motors 3 induziert. Diese würden das Magnetfeld im *Field of View,* welches sich im Untersuchungsbereich befindet, stören und/oder den Motor 3 unzulässig erwärmen oder auf andere Weise in seiner Funktionsweise behindern. Zudem könnte eine Platzierung zu nah am *Field of View* bzw. am Untersuchungsbereich dazu führen, dass der Motor 3 mit seinem (sich drehenden) Magnetfeld Schwankungen des Magnetfelds am Ort des *Field of View* verursacht, welche die Messungen stören. Ein weiterer Nachteil dieser Ausführung gemäß dem Stand der Technik ist es, dass, um einen ausreichend starken Luftstrom in dem gesamten Tunnel erzeugen zu können, um stickige Luft zu vermeiden, eine relativ starke Strömungsgeschwindigkeit am Luftausgang 5 benötigt wird. Das kann für einen Patienten 30 unangenehm sein, wenn eine Körperregion (z.B. Brustkorb, Zwerchfell) untersucht werden soll, deren Positionierung im *Field of View* dazu führt, dass der Kopf des Patienten 30 direkt in der Nähe des Luftausgang 5 zu liegen kommt, wo der Luftstrom besonders stark ist.

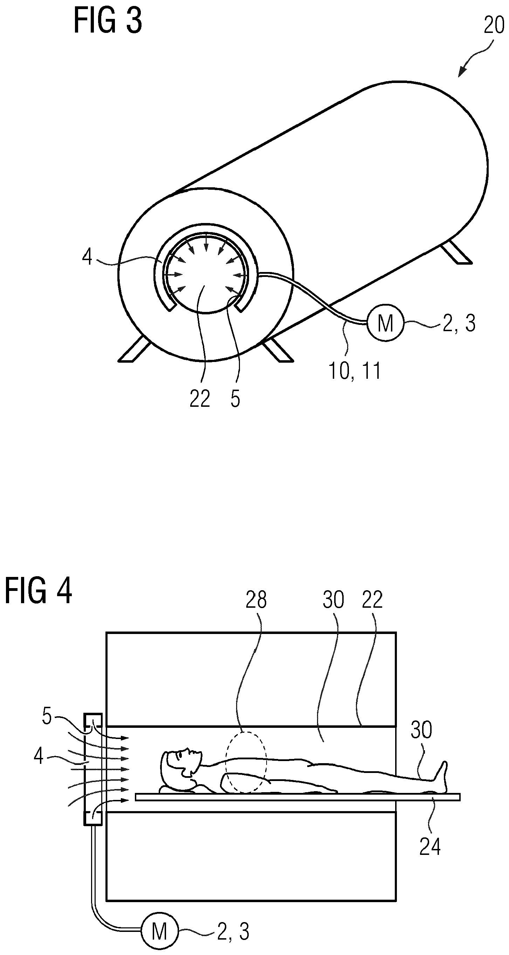

Figur 3 und 4 zeigen ein Magnetresonanztomographiesystem gemäß einer Ausführungsform der Erfindung in einer Perspektivansicht (Figur 3) und in einer seitlichen Schnittansicht (Figur 4). Der Magnetresonanztomograph 20 weist einen Untersuchungstunnel 22 auf, in dem sich ein Untersuchungsbereich befindet und an dessen Tunneleingang ein erfindungsgemäßer Luftmengenverstärker 4 angeordnet ist. In dem Untersuchungsbereich befindet sich das *Field of View* 28, also der Bereich, in dem der Magnetresonanztomograph ein Bild erstellen kann, was hier etwa eine Kugel mit 40cm Durchmesser ist, die in der Mitte des Untersuchungstunnels liegt. In der Darstellung von Figur 4 kann ein Bild vom Brustkorb eines Patienten 30 gemacht werden.

Der Luftmengenverstärker 4 ist hier um die hintere Tunnelöffnung herum angeordnet und so ausgerichtet, dass der durch ihn verstärkte Luftstrom in den Untersuchungstunnel 22 gerichtet ist. Der Luftmengenverstärker 4 ist entlang einem Rand des Tunneleingangs angeordnet und ist entsprechend der Form des Tunneleingangs ringförmig ausgestaltet, wobei dieser Ring im unteren Bereich unterbrochen ist, sodass der Luftmengenverstärker 4 den Tunneleingang teilringförmig umgibt. Der offen gelassene Teil des Ringumfangs kann verwendet werden, um Platz für weitere Komponenten, insbesondere Anschlussleitungen der Gradientenspule zu gewähren. Alternativ kann der Luftmengenverstärker als vollständiger Ring ausgeprägt sein, wodurch eine Luftmengenverstärkung und eine gleichmäßige Luftströmung noch effizienter sein kann.

Ein Luftstromerzeuger 2 mit einem Motor 3 ist in einem Bereich angeordnet, in dem beim Betrieb des MRT 20 das Magnetfeld relativ niedrig, insbesondere niedriger als am Tunneleingang, ist. Zusätzlich kann der Luftstromerzeuger an einem Ort platziert werden, an dem weniger Konkurrenz mit verschiedenen anderen Komponenten, insbesondere mit Elektronickomponenten, die seitlich und gut zugänglich am Magneten untergebracht werden sollen, um den begrenzt vorhandenen Bauraum herrscht. Der Luftstromerzeuger 2 umfasst vorzugsweise einen für einen höheren Differenzdruck bei geringerem Luftdurchsatz ausgelegte Ventilator, z.B. einen Diagonal- oder Radiallüfter.

Von dem Luftstromerzeuger 2 erzeugte Luft wird durch einen Luftleitungsabschnitt 10, insbesondere einem Luftschlauch 11, zu dem Luftmengenverstärker geleitet. Weil nur ein Bruchteil, z.B. ca. 1/15 des am Luftausgang 5 ausgegebenen Gesamt-Luftstroms, durch den Luftleitungsabschnitt 10, befördert werden muss, kann dieser länger und dünner ausfallen. Beispielsweise kann der Luftleitungsabschnitt 10 2-3m lang sein und einen Durchmesser von 40-50mm aufweisen.

Der Luftstrom aus dem Luftmengenverstärker 4 bleibt in dieser Ausführungsform eher nahe der Wand des Untersuchungstunnels 22, während weiter innen die beim Verstärken des Luftstroms mitgerissene Luft bewegt wird. Dabei gibt es an keiner Stelle des Tunnelquerschnitts eine Luftströmung, die deutlich schneller ist als an anderen Stellen. Lokale Bereiche größerer Luftströmung, die dem Patienten 30 unangenehm ins Gesicht blasen könnten, können so vorteilhafterweise vermieden werden. Gleichzeitig kann eine besonders gleichmäßige Belüftung gewährleistet werden. Dies kann insbesondere vorteilhaft sein, da der Untersuchungstunnel in der Regel relativ eng ausgebildet ist, z.B. im Bereich von 60-80cm Querschnittsdurchmesser, und gegebenenfalls nicht viel Platz für die Lüftung bleibt, wenn ein Patient 30 in dem Untersuchungstunnel 22 liegt. Gerade bei längeren Untersuchungszeiten (z.B. bis zu 20 Minuten) ist daher eine gute Lüftung wünschenswert, die mit dieser Ausführungsform besonders effizient ermöglicht wird.

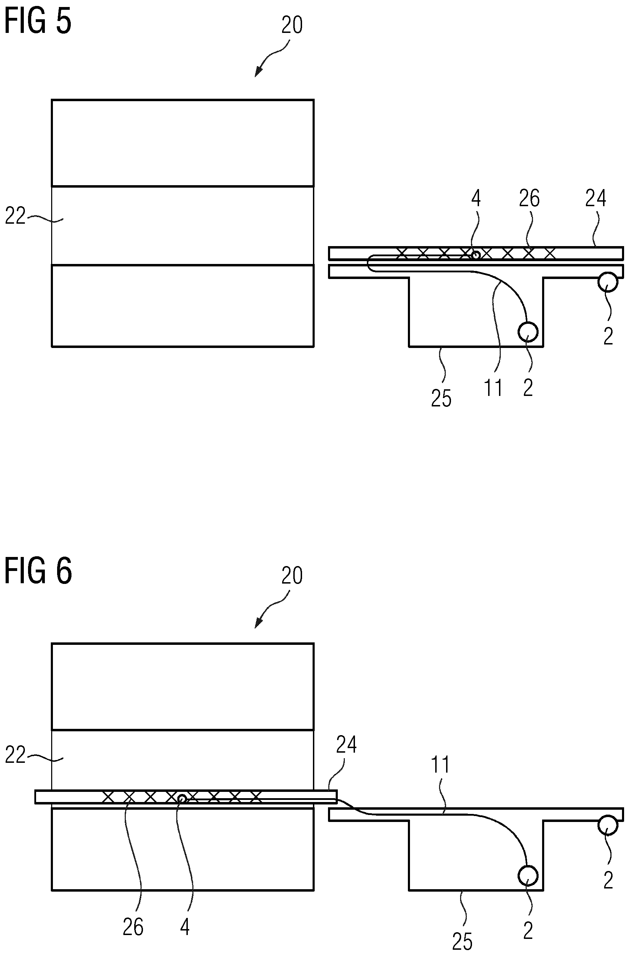

Figur 5 zeigt eine Schnittansicht auf ein Magnetresonanztomographiesystem mit ausgefahrener Patientenliege 24 gemäß einer weiteren Ausführungsform der Erfindung, während Figur 6 eine Schnittansicht auf das Magnetresonanztomographiesystem mit eingefahrener Patientenliege 24 zeigt. Der Durchmesser des Untersuchungstunnels kann beispielsweise im Bereich von 60-80cm liegen. Die Länge des MRT-Magneten kann beispielsweise kleiner als 2m sein.

Die Patientenliege 24 ist dazu ausgestaltet, in einen Untersuchungsbereich im Untersuchungstunnel 22 des MRT-Systems bewegt zu werden. Die Patientenliege 24 ist verfahrbar, damit sich ein Patient 30 außerhalb des Untersuchungstunnels auf die Patientenliege 24 legen kann (Figur 5) und dann in den Tunnel des Magneten gefahren werden kann (Figur 6). Der Luftmengenverstärker 4 ist in dieser Ausführungsform an der Patientenliege 24 befestigt, sodass er, wenn die Patientenliege 24 in den Untersuchungstunnel 22 bewegt wird, mit der Patientenliege 24 in den Untersuchungstunnel 22 bewegt wird.

Die Patientenliege umfasst mehrere Empfangsspulen 26, die in der Patientenliege verbaut sind, und der Luftmengenverstärker 4 ist so angeordnet, dass der von ihm ausgegebene verstärkte Luftstrom zu einem überwiegenden Teil auf die Empfangsspulen 26 gerichtet ist. Die Empfangsspulen 26 dienen insbesondere dazu, Bilder vom Rückenbereich des Patienten 30 (genannt *"spine coils")* anzufertigen. Die ungefähre Position der Empfangsspulen ist mit Kreuzen markiert. Nahe an den Empfangsspulen 26 sind jeweils Verstärker verbaut *(low noise amplifier* = LNA). Im Stand der Technik muss bisher auf Luftkonvektion für die Kühlung der Spulen und der Verstärker gesetzt werden, da ein Lüften im Untersuchungstunnel 22 nicht möglich war. Alternativ ist es auch denkbar andere, z.B. bisher mangels ausreichender Kühlung nicht verwendbare, Komponenten, insbesondere andere Elektronikkomponenten, zu verbauen, die mit dem adaptierten Lüfter gekühlt werden können. Elektronische Komponenten können beispielsweise mit kleineren Kühlkörpern versehen sein und/oder leistungsfähiger als bisher ausgestaltet sein, was insbesondere ermöglicht wird, da sie mittels des adaptierten Lüfters mit einem Luftstrom, statt nur über Konvektion, gekühlt werden können.

Vor dem Tunneleingang des Untersuchungstunnels 22 ist ein Stützelement 25 angeordnet, durch das die Patientenliege 24 durch Aufliegen auf dem Stützelement 25 gestützt wird, soweit sie außerhalb des Untersuchungstunnel ist (gemäß Figur 5). Der Luftstromerzeuger 2 ist in dieser Ausführungsform außen an dem Stützelement 25 angeordnet oder in das Stützelement 25 integriert. In dieser Darstellung sind zwei bevorzugte Positionierungen für den Luftstromerzeuger 2 eingezeichnet, in denen der Luftstromerzeuger jeweils alternativ oder, insbesondere bei mehreren adaptierten Lüftern gleichzeitig, angeordnet sein könnte. In den gezeigten Positionen am Fuß des Stützelements ist das von dem großen MRT-Magneten erzeugte Streufeld gering genug, um einen konventionellen Lüftermotor 3 verwenden zu können.

Der Luftleitungsabschnitt 10 ist in diesem Beispiel ein flexibler Luftschlauch 11. Es kann beispielsweise vorgesehen sein, dass der Luftschlauch 11 zusammen mit anderen Kabeln, die vom unbeweglichen in den beweglichen Teil, d.h. von dem Stützelement 25 zu der Patientenliege 24, laufen, geführt wird. Der Luftschlauch kann beispielsweise eine Länge von ca. 3m und einen maximalen Durchmesser von ca. 3cm aufweisen. Ein größerer Durchmesser würde möglicherweise erfordern, dass gegenüber einem bisher üblichen Design wesentliche Änderungen an der Mechanik des Tisches bzw. der Patientenliege 24 vorgenommen werden müssten.

Der Luftmengenverstärker 4 ist vorzugsweise an die Maße der Patientenliege 24 bzw. den Bereich der Patientenliege, in dem die zu belüftenden Elektronikkomponenten sind, angepasst. Beispielsweise kann der ringförmige Luftmengenverstärker 4 einen Außendurchmesser von ca. 5cm aufweisen.

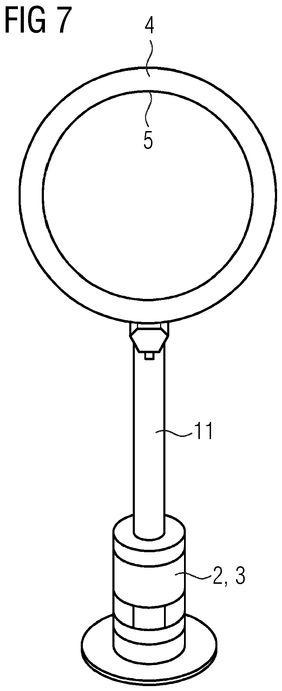

Figur 7 zeigt eine Ansicht auf einen adaptierten Lüfter gemäß einer Ausführungsform der Erfindung. Der adaptierte Lüfter umfasst einen Luftstromerzeuger 2 mit einem Motor 3 und einen ringförmigen Luftmengenverstärker 4, der einen Luftausgang 5 des adaptierten Lüfters umfasst. Weiterhin umfasst der adaptierte Lüfter einen Luftleitungsabschnitt 10, der einen durch den Luftstromerzeuger 2 erzeugten Luftstrom zu dem Luftmengenverstärker 4 leitet, wo dieser Luftstrom von dem Luftmengenverstärker 4 verstärkt wird, sodass ein Luftstrom mit vergrößerter Luftmenge bzw. ein verstärkter Luftstrom erzeugt wird. Der Luftmengenverstärker 4 ist bevorzugt aus einem Kunststoff gefertigt und weist keine beweglichen Teile auf.

Der Luftstrom wird im Wesentlichen durch die Geometrie des Luftmengenverstärkers 4 und des Luftausgangs 5 verstärkt.

## Patentansprüche

1. Magnetresonanztomographiesystem umfassend einen adaptierten Lüfter mit einem Luftausgang (5),
wobei der adaptierte Lüfter
a. einen Luftstromerzeuger (2), insbesondere mit einem Motor (3),
b. einen Luftmengenverstärker (4), der an dem Luftausgang (5) angeordnet ist und/oder der den Luftausgang (5) umfasst, und
c. einen Luftleitungsabschnitt (10), der einen durch den Luftstromerzeuger (2) erzeugten Luftstrom zu dem Luftmengenverstärker (4) leitet, umfasst,
wobei der Luftmengenverstärker (4) derart ausgestaltet ist, dass er eine Luftmenge des über den Luftleitungsabschnitt (10) zu dem Luftmengenverstärker (4) geleiteten Luftstroms vergrößert, sodass er einen Luftstrom mit vergrößerter Luftmenge ausgibt.

2. Magnetresonanztomographiesystem gemäß Anspruch 1,
wobei der Luftmengenverstärker (4) aus einem nicht-magnetischen bzw. nicht-magnetisierbaren Material und/oder einem nicht-leitfähigen Material, insbesondere aus einem Kunststoff, gefertigt ist.

3. Magnetresonanztomographiesystem gemäß einem der vorhergehenden Ansprüche,
wobei der Luftmengenverstärker (4) derart ausgestaltet ist, dass er durch seine geometrische Gestaltung die Luftmenge passiv vergrößert.

4. Magnetresonanztomographiesystem gemäß einem der vorhergehenden Ansprüche,
wobei der Luftmengenverstärker (4) einen luftführenden Ring oder Ringabschnitt mit Luftausgangsöffnungen aufweist, durch welche Luft in eine Richtung ausgegeben wird, die eine Richtungskomponente senkrecht zu der Ringebene aufweist, insbesondere in eine Richtung senkrecht zur Ringebene oder in eine um maximal 20° von der Richtung senkrecht zur Ringebene abweichenden Richtung ausgegeben wird.

5. Magnetresonanztomographiesystem gemäß einem der vorhergehenden Ansprüche,
wobei der Luftstromerzeuger (2) in einem ersten Bereich angeordnet ist, der, wenn das Magnetresonanztomographiesystem während des Betriebs ein Magnetfeld erzeugt, eine geringere magnetische Feldstärke aufweist als ein zweiter Bereich, in dem der Luftmengenverstärker (4) angeordnet ist.

6. Magnetresonanztomographiesystem gemäß einem der vorhergehenden Ansprüche,
wobei der Luftmengenverstärker (4) dazu ausgestaltet ist, die Luftmenge um zumindest das 5-fache, bevorzugt zumindest das 10-fache, besonders bevorzugt zumindest das 15-fache, zu verstärken.

7. Magnetresonanztomographiesystem gemäß einem der vorhergehenden Ansprüche,
wobei der Luftstromerzeuger (2) dazu ausgestaltet ist, den Luftstrom in den Luftleitungsabschnitt (10) mit einem Druck von 80 bis 500 Pa, bevorzugt, 100 bis 350 Pa, besonders bevorzugt von 150 Pa bis 300 Pa einzugeben.

8. Magnetresonanztomographiesystem gemäß einem der vorhergehenden Ansprüche,
wobei das Magnetresonanztomographiesystem einen Untersuchungstunnel (22) mit einem Tunneleingang umfasst, wobei der Luftmengenverstärker (4) an dem Tunneleingang angeordnet ist und so ausgerichtet ist, dass der verstärkte Luftstrom in den Untersuchungstunnel (22), insbesondere im Wesentlichen entlang der Längsachse des Untersuchungstunnels (22), gerichtet ist, oder so ausgerichtet ist, dass der verstärkte Luftstrom aus dem Untersuchungstunnel herausgerichtet ist.

9. Magnetresonanztomographiesystem gemäß Anspruch 8,
wobei der von dem Luftmengenverstärker (4) umfasste Luftausgang (5) entlang einem Rand des Tunneleingangs angeordnet ist und insbesondere den Tunneleingang ringförmig oder teilringförmig umgibt.

10. Magnetresonanztomographiesystem gemäß einem der vorhergehenden Ansprüche,
wobei der von dem Luftmengenverstärker (4) umfasste Luftausgang (5) ringförmig oder in Form eines Ringabschnitts ausgestaltet ist.

11. Magnetresonanztomographiesystem gemäß einem der vorhergehenden Ansprüche,
wobei das Magnetresonanztomographiesystem eine Patientenliege (24) umfasst, die dazu ausgestaltet ist, zumindest teilweise in einen Untersuchungsbereich des Magnetresonanztomographiesystems bewegt zu werden,
wobei der Luftmengenverstärker (4) an der Patientenliege (24) angeordnet und befestigt ist, sodass er, wenn die Patientenliege (24) in den Untersuchungsbereich bewegt wird, mit der Patientenliege (24) in den Untersuchungsbereich bewegt wird.

12. Magnetresonanztomographiesystem gemäß Anspruch 11,
wobei die Patientenliege (24) zumindest eine elektronische Komponente, insbesondere zumindest eine Empfangsspule (26), umfasst,
wobei der Luftmengenverstärker (4) derart angeordnet ist, dass der ausgegebene verstärkte Luftstrom zu einem überwiegenden Teil auf die zumindest eine elektronische Komponente gerichtet ist.

13. Magnetresonanztomographiesystem gemäß Anspruch 11 oder 12,
wobei das Magnetresonanztomographiesystem einen Untersuchungstunnel (22) mit einem Tunneleingang, über den die Patientenliege (24) in den Untersuchungsbereich bewegbar ist, umfasst, wobei der Untersuchungsbereich in dem Untersuchungstunnel (22) angeordnet ist,
wobei das Magnetresonanztomographiesystem ein vor dem Tunneleingang angeordnetes Stützelement (25) umfasst, wobei die Patientenliege (24) durch Aufliegen auf dem Stützelement (25) gestützt wird, soweit sie außerhalb des Untersuchungstunnel (22) bewegt wird,
wobei der Luftstromerzeuger (2) außen an dem Stützelement (25) angeordnet ist oder in das Stützelement (25) integriert ist,
wobei der Luftleitungsabschnitt (10) zwischen dem Luftstromerzeuger (2) und dem Luftmengenverstärker (4) flexibel, insbesondere als Luftschlauch (11), ausgebildet ist.

14. Verwendung eines Luftmengenverstärkers (4), insbesondere eines Luftmengenverstärkers (4) wie beschrieben in den vorhergehenden Ansprüchen, in und/oder an einem Magnetresonanztomographen, insbesondere zur Erzeugung eines verstärkten Luftstroms und/oder zur Kühlung zumindest einer Komponente des Magnetresonanztomographen.

15. Verwendung eines adaptierten Lüfters, insbesondere eines adaptierten Lüfters wie beschrieben in den Ansprüchen 1 bis 13, zur Kühlung zumindest einer Komponente eines Magnetresonanztomographen (20) und/oder zur Belüftung eines Untersuchungsbereichs des Magnetresonanztomographen,
wobei der adaptierte Lüfter
einen Luftstromerzeuger (2) mit einem Motor (3),
einen Luftmengenverstärker (4), der an dem Luftausgang (5) angeordnet ist und/oder der der Luftausgang (5) ist,
und
einen Luftleitungsabschnitt (10), der einen durch den Luftstromerzeuger (2) erzeugten Luftstrom zu dem Luftmengenverstärker (4) leitet, umfasst,
wobei der Luftmengenverstärker (4) derart ausgestaltet ist, dass er eine Luftmenge des über den Luftleitungsabschnitt (10) zu dem Luftmengenverstärker (4) geleiteten Luftstroms vergrößert, sodass er einen Luftstrom mit vergrößerter Luftmenge ausgibt.
